# EUROPEAN PATENT APPLICATION

(11) **EP 4 042 868 A1**
(43) Date of publication of application: **17.08.2022**
(21) Application number: 20874759.2
(22) Date of filing: 08.10.2020
(51) Int. Cl.: A01N 1/02, C12N 5/07, C12N 1/04

(54) **COMPOSITION FOR FREEZING CELLS, METHOD FOR FREEZING CELLS, METHOD FOR CULTURING CELLS, AND KIT FOR FREEZING CELLS**

(30) Priority: 08.10.2019 JP 2019185161
(71) Applicant: Iwatani Corporation, Osaka 541-0053 (JP)
(72) Inventor: TABATA Yasuhiko, Uji-shi, Kyoto 611-0024 (JP); KIMURA Koji, Amagasaki-shi, Hyogo 661-0965 (JP)
(74) Representative: Mewburn Ellis LLP
(86) International application number: PCT/JP2020/038136
(87) International publication number: WO 2021/070908

(57) **Abstract**

A cell freezing composition according to the present invention includes a medium, an antifreezing agent, and dissolved hydrogen.

## Description

### TECHNICAL FIELD

The present invention relates to a cell freezing composition, a cell freezing method, a cell culture method, and a cell freezing kit.

### BACKGROUND ART

Various cell freezing techniques have been developed until now. As such type of techniques, a technique described in Patent Document 1, for example, is known. Patent Document 1 describes a freezing-preservation medium composed of 90% of fatal bovine serum and 10% of DMSO (Patent Document 1, Paragraph [0073] and the like) .

### RELATED DOCUMENT

### PATENT DOCUMENT

[Patent Document 1] Japanese Laid-Open Patent Application No. 2012-512637

### SUMMARY OF THE INVENTION

However, the present inventor discovered as a result of study that there is room for improvement in the freeze-preservation medium described in Patent Document 1 in terms of cell proliferation ability after thawing.

### Means to solve problems

In a typical cell freezing method, a cell freezing preservation solution containing an antifreezing agent such as DMSO is used in a cell culture medium. Cells are placed in the cell freezing preservation solution and are then frozen and preserved. The frozen and preserved cells are thawed, cultured, and proliferated to a large amount. If the cells are damaged by a low-temperature stress or the like at the time of freezing and thawing the cells in this ordinary method, cell survival activity, cell proliferation speed, and the like may be reduced in the cells after the thawing, and the cell proliferation ability may be degraded.

The present inventor discovered as a result of intensive study regarding an environment in a cell freezing preservation solution on the basis of the aforementioned circumstances that the degradation of the cell proliferation ability is curbed in the cells after thawing by adding hydrogen water and/or hydrogen gas to the cell freezing preservation solution and causing the preservation solution to contain dissolved hydrogen and completed the present invention.

According to the present invention, there is provided a cell freezing composition including: a medium; an antifreezing agent; and dissolved hydrogen.

Also, according to the present invention, there is provided a cell freezing composition including: a medium; and an antifreezing agent, in which a redox potential of the cell freezing composition measured at 25°C under an atmospheric pressure is equal to or greater than -700 mV and equal to or less than -100 mV, and pH of the cell freezing composition measured at 25°C under an atmospheric pressure is equal to or greater than 6.8 and equal to or less than 8.5.

Also, according to the present invention, there is provided a cell freezing method including: preparing a mixture of the cell freezing composition and cells; and freezing the mixture to obtain a frozen article.

Also, according to the present invention, there is provided a cell culture method including: thawing a frozen article obtained by the cell freezing method and obtaining a thawed article; and culturing cells contained in the obtained thawed article.

Moreover, according to the present invention, there is provided a cell freezing kit including a preservation container containing hydrogen water.

According to the present invention, there is provided a cell freezing composition with excellent cell proliferation ability after thawing, a cell freezing method, a cell culture method, and a cell freezing kit using the same.

### BRIEF DESCRIPTION OF THE DRAWINGS

Fig. 1 is a diagram illustrating MTT activity values per cell in Example 1 and Comparative Example 1.
Fig. 2 is a diagram illustrating cell proliferation curves in Example 1 and Comparative Example 1.
Fig. 3 is a diagram illustrating MTT activity values in Examples 3 to 5 and Comparative Example 2.

### DESCRIPTION OF EMBODIMENTS

An outline of a cell freezing composition according to the present embodiment will be described.

One cell freezing composition according to the present embodiment includes a medium, an antifreezing agent, and dissolved hydrogen.

According to a knowledge of the present inventor, it was discovered that it was possible to increase a cell survival activity and cell proliferation speed in cells after thawing after cells contained in the cell freezing composition are frozen, preserved, and thawed by adding hydrogen water and/or hydrogen gas to a cell freezing composition containing a cell culture medium containing cell nutrients and an antifreezing agent and causing the cell freezing composition to contain dissolved hydrogen and thereby to curb degradation of cell proliferation ability.

The dissolved hydrogen means hydrogen molecules dissolved in the solution. A case in which dissolved hydrogen concentration of 0.1 ppm or more, for example, at 25°C under an atmospheric pressure is shown using a dissolved hydrogen meter is assumed to be a state in which dissolved hydrogen is contained.

Although detailed mechanisms are not clear, it is considered to be possible to promote activity of cells at the time of mixing before a freezing operation if the concentration of the dissolved hydrogen in the cell freezing composition becomes relatively high and thereby to reduce damage on the cells in the process of the freezing. Also, an effect of reactive oxygen removal ability of the dissolved hydrogen is also conceivable as a factor for reducing damage on the cells.

It is possible to realize a cell proliferation ability after thawing and to realize a cell freezing method capable of obtaining cells with excellent MTT activity and a cell culture method using the cells, by using the cell freezing composition according to the present embodiment. Also, it is possible to realize a cell freezing method and a cell culture method with an excellent cell proliferation speed.

Such a method is a method use that can be used to preserve and transport organs and that is useful for medical techniques such as regenerative medicine.

Hereinafter, each component of the cell freezing composition according to the present embodiment will be described in detail.

The cell freezing composition is a hydrogen-containing cell freezing preservation solution used to freeze cells.

The cells include at least one or more of a single cell, a plurality of cells, a tissue as aggregated cells, and an organ (body part).

As the cells used in the cell freezing, it is possible to use animal cells or plant cells, for example.

Examples of the animal cells include invertebrate derived cells such as insect cells and vertebrate-derived cells such as mammalian-derived cells such as human cells and mouse cells, bird-derived cells, amphibian-derived cells, reptile-derived cells, and fish-derived cells. Among these, mammalian-derived cells may be used.

Examples of the mammals include primates such as humans, mice, guinea pigs, rats, cows, horses, pigs, goats, dogs, rabbits, and the like.

The animal cells may be cells in any forms such as primary cultured cells, established cells, passaged cells, cultured cells, body cells, stem cells, tumor cells, and the like. The stem cells include adult stem cells, ES cells, and iPS cells.

More specific examples of the cells include ES cells, iPS cells, mesenchymal cells, fibroblast cells, muscle cells, bone cells, nerve cells, epithelial cells, and stem cells or progenitor cells thereof, and cells differentially induced therefrom. The cells may be adherent cells or floating cells.

The cell freezing composition contains a medium. The medium can include components necessary to maintain cell life.

Although the medium is not particularly limited as long as the medium contains cell nutrients, examples thereof include a freezing medium, a complete medium, a serum-containing medium, a serum-free medium, and the like. In a case of cells used for regenerative medicine, a serum-free medium may be used.

As specific examples of the medium, known basal media may be used, including a DMEM medium, an EMEM medium, an RPMI-1640 medium, an α-MEM medium, an F-12 medium, an F-10 medium, an M-199 medium, an HAM medium, an ERDF medium, an L-15 medium, a Williams medium, and the like. Among these, one kind may be used alone, or two or more kinds may be used in combination.

The cell freezing composition contains an antifreezing agent. It is thus possible to protect the cells from cell damage due to ice crystals and an osmotic pressure action.

The antifreezing agent is not particularly limited, a known antifreezing agent may be used, and specific examples thereof include dimethyl sulfoxide (DMSO), hydroxyethyl starch (HES), ethylene glycol (EG), glycerol, and the like. Among these, one kind may be used alone, or two or more kinds may be used in combination.

The concentration of the antifreezing agent is appropriately selected in accordance with the type of the antifreezing agent, a cell type, and the like and may be 1% (v/v) to 15% (v/v) and is more preferably 5% (v/v) to 10% (v/v), for example, with respect to the entire medium.

The cell freezing composition may be configured to contain hydrogen water.

The hydrogen water is water with hydrogen dissolved therein and means water with a predetermined or higher dissolved hydrogen concentration at 25°C under an atmospheric pressure.

The hydrogen water is prepared and obtained using raw water and using a pressure dissolution scheme, a spiral flow scheme, an ultrasonic scheme, a micropore scheme, or the like. Among these, the pressure dissolution scheme may be used. It is possible to appropriately control the concentration of dissolved hydrogen and the concentration of dissolved oxygen by appropriately selecting a scheme. For example, it is possible to boost the concentration of the dissolved hydrogen and to reduce the concentration of dissolved oxygen in water by the pressure dissolution scheme.

As raw water, distilled water, ion exchanged water, ultrapure water, or the like may be used. The water sterilized through heating treatment, filter treatment, or the like can be used.

A lower limit of the concentration of dissolved hydrogen in hydrogen water measured at 25°C under an atmospheric pressure may be equal to or greater than 0.1 ppm, is preferably equal to or greater than 0.2 ppm, and is more preferably equal to or greater than 0.3 ppm, for example. On the other hand, the concentration of dissolved hydrogen in raw water placed in an open environment such that it comes into contact with the atmospheric air is typically 0.0 ppm at 25°C under an atmospheric pressure. On the other hand, an upper limit of the concentration of dissolved hydrogen in hydrogen water is equal to or less than 10.0 ppm, is equal to or less than 5.0 ppm, is preferably equal to or less than 2.0 ppm, is equal to or less than 1.6 ppm, and is equal to or less than 1.5 ppm, for example.

An upper limit of the concentration of dissolved oxygen in hydrogen water measured at 25°C under an atmospheric pressure is equal to or less than 9.0 ppm, is more preferably equal to or less than 5.0 ppm, and is more preferably equal to or less than 3.0 ppm, for example. On the other hand, a lower limit of the concentration of dissolved oxygen in hydrogen water is not particularly limited and may be equal to or greater than 0.0 ppm.

Although the hydrogen water may contain fine bubbles such as ultrafine bubbles of equal to or less than 1 µm or microbubbles of greater than 1 µm and equal to or less than 100 µm, the hydrogen water may be configured not to contain such fine bubbles.

The cell freezing composition may contain any one or more of appropriate serums and additives as needed within ranges that do not damage effects of the present invention, in addition to the aforementioned components.

Examples of the additives include an amino acid, a non-essential amino acid, vitamins, a low-molecular compound, a sugar, a protein, a buffer, inorganic salts, an antibiotic, an antibacterial agent, an antioxidant, an antifreezing agent, and the like. Among these, one kind may be used alone, or two or more kinds may be used in combination.

As a serum, an animal serum may be used, for example, and specific examples include a human serum, a fatal bovine serum (FBS), a bovine serum, a calf serum, a goat serum, a horse serum, a pig serum, a sheep serum, a rabbit serum, a rat serum, and the like.

Examples of the buffer include PBS, HEPES, MES, HANK'S, and the like.

Although a method of preparing the cell freezing composition is not particularly limited, the cell freezing composition can be obtained by mixing each of the aforementioned components. The order of adding the components is appropriately changed and is not limited. For example, the hydrogen water, the medium, and the antifreezing agent may be mixed, or the antifreezing agent may be added to a mixture solution of a hydrogen water and a medium produced in advance.

Alternatively, a medium with hydrogen gas added thereto in advance may be mixed with other components, or hydrogen gas may be added to a mixture of the medium and other components.

The cell freezing composition obtained as described above has the following characteristics.

An upper limit of the redox potential of the cell freezing composition measured at 25°C under an atmospheric pressure is equal to or less than -100 mV, is preferably equal to or less than -150 mV, and is more preferably equal to or less than -200 mV, for example. In this manner, a cell proliferation ability after the thawing can be enhanced. On the other hand, a lower limit of the redox potential of the cell freezing composition may be equal to or greater than -700 mV and equal to or greater than -600 mV, for example.

A lower limit of the concentration of dissolved hydrogen in the cell freezing composition measured at 25°C under an atmospheric pressure is equal to or greater than 0.1 ppm, is preferably equal to or greater than 0.2 ppm, and is more preferably equal to or greater than 0.3 ppm, for example. In this manner, a cell proliferation ability after the thawing can be enhanced. On the other hand, an upper limit of the concentration of dissolved hydrogen in the cell freezing composition is equal to or less than 10.0 ppm, is equal to or less than 5.0 ppm, is more preferably equal to or less than 2.0 ppm, is equal to or less than 1.6 ppm, and may be equal to or less than 1.5 ppm.

An upper limit of the concentration of the dissolved oxygen in the cell freezing composition measured at 25°C under an atmospheric pressure is equal to or less than 9.0 ppm, is more equal to or less than 5.0 ppm, and is more preferably equal to or less than 3.0 ppm, for example. In this manner, a cell proliferation ability after the thawing can be enhanced. On the other hand, a lower limit of the concentration of dissolved oxygen in the cell freezing composition is not particularly limited and may be equal to or greater than 0.0 ppm.

A lower limit of pH of the cell freezing composition measured at 25°C under an atmospheric pressure is equal to or greater than 6.8 and is preferably equal to or greater than 7.0, for example. On the other hand, an upper limit of pH of the cell freezing composition is equal to or less than 8.5 and is preferably equal to or less than 8.4, for example. It is possible to realize an environment appropriate for culturing cells by setting pH within the aforementioned numerical range.

Also, as another cell freezing composition according to the present embodiment, a cell freezing composition including: a medium; and an antifreezing agent, in which a redox potential of the cell freezing composition measured at 25°C under an atmospheric pressure is equal to or greater than -700 mV and equal to or less than -100 mV, and pH of the cell freezing composition measured at 25°C under an atmospheric pressure is equal to or greater than 6.8 and equal to or less than 8.5 can be exemplified.

In regard to the numerical values of the redox potential and pH of the cell freezing composition at this time, the aforementioned upper limits and lower limits may be appropriately used in combination.

According to the present embodiment, it is possible to curb degradation of cell proliferation ability after thawing for the cells thawed using the cell freezing composition by appropriately preparing the numerical range of the redox potential and the numerical range of pH of the cell freezing composition.

In the present embodiment, it is possible to control the aforementioned redox potential, the concentration of dissolved hydrogen, the concentration of dissolved oxygen, and pH by appropriately selecting a type and a blending amount of each component contained in the cell freezing composition, a method for preparing the cell freezing composition. Among these, examples of elements for causing the redox potential, the concentration of dissolved hydrogen, the concentration of dissolved oxygen, and pH to fall within desired numerical ranges include using hydrogen water with the concentration of dissolved hydrogen in a supersaturated state, a saturated state, and a state close to a saturated state, appropriately adjusting a hydrogen water storage condition, a time until addition of cells after preparation of the cell freezing composition, and the like.

The redox potential, the concentration of dissolved hydrogen, the concentration of dissolved oxygen, and pH of the cell freezing composition are preferably values measured at an ordinary pressure (23°C) in an ordinary pressure and values immediately before utilization in cell freezing. "Immediately before" means within 1 hour, preferably within 30 minutes, and more preferably within 15 minutes, for example.

Particularly, the concentration of dissolved hydrogen is preferably a value immediately before utilization in cell freezing since the concentration gradually decreases with time if the hydrogen water/cell freezing composition is in an open environment.

Although the saturated hydrogen concentration of water at an ordinary temperature in an ordinary pressure is 1.6 ppm, it is possible to prepare the hydrogen water/cell freezing composition with concentration of dissolved hydrogen in a supersaturated state by adding hydrogen gas to water and the medium under a pressurized condition.

The addition of hydrogen gas can be performed in a container such as an aluminum pouch, a bag prepared of a resin, or a box prepared of resin, for example, under a pressure. It is possible to prepare the hydrogen water and the cell freezing composition with no influences on frozen cells and an operator (human) by setting the concentration of dissolved hydrogen to be equal to or less than the aforementioned upper limit value. Also, it is possible for the operator to prepare the hydrogen water and the cell freezing composition with prescribed concentration of dissolved hydrogen near a site of the cell freezing operation.

It is possible for the operator to prepare the hydrogen water and the cell freezing composition with prescribed concentration of dissolved hydrogen near the operation site immediately before cell freezing.

Also, the cell freezing composition may be prepared using hydrogen water manufactured in another facility and stored and transported in a preservation container.

A cell freezing method and a cell culture method according to the present embodiment will be described.

As devices and instruments used for freezing, storing, and cooling cells and various reagents and components used therewith, sterilized ones may be used as needed.

The cell freezing method includes: preparing a mixture of the aforementioned cell freezing composition and cells; and freezing the mixture to obtain a frozen article. The mixture can be obtained by suspending the cells in the cell freezing composition, for example.

The cells may be obtained by collecting subcultured cells, for example. Adherent cells can be collected as sediment by peeling off the adherent cells from the surface of the cell container and performing centrifuging in a predetermined medium. Floating cells can be collected as sediment by centrifuging a suspension in a culture container.

Although the density of the seeded cells differs depending on the type and the like of the cells, the density may be about 1 × 10³ cells/mL to about 1 × 10⁹ cells/mL and may be more preferably about 1×10⁴ cells/mL to 1 × 10⁷ cells/mL, for example.

The cell freezing may be performed in accordance with a protocol for freezing the cells.

An example of the cell freezing protocol will be shown below.
(1) Cells are suspended in a culture medium, and the suspension is placed in a 15 mL conical tube.
(2) Serum and dimethyl sulfoxide (DMSO) are added such that the cell suspension, the serum, and dimethyl sulfoxide meet the proportion of 7:2:1.
(3) 1 mL of mixture solution obtained in (2) above is dispensed to a cryotube.
(4) The cells are frozen and preserved.

In the aforementioned protocol, hydrogen water may be added to the suspension in the procedure (1) or may be added along with the serum and the like in the procedure (2). Since it is easy to adjust the concentrations of each component, the serum, and DMSO in the suspension, it is possible to add the hydrogen water in the procedure (1) .

Although the method for preparing the cell freezing composition is not particularly limited, it is possible to use at least one of a method of mixing hydrogen water into a medium, a method of adding hydrogen water to an antifreezing agent, a method of adding hydrogen water to a mixture solution containing the medium and the antifreezing agent, a method of adding hydrogen gas to the medium, a method of adding hydrogen gas to the antifreezing agent, and a method of adding hydrogen gas to a mixture solution containing the medium and the antifreezing agent.

In the case of the method of mixing the hydrogen water, components such as the hydrogen water, the medium, and the antifreezing agent may be mixed, or the components such as the antifreezing agent may be added to a mixture solution of the hydrogen water and the medium produced in advance.

In the case of the method of adding hydrogen gas, a mixture obtained by adding hydrogen gas to each component such as the medium and the antifreezing agent and the other components may be mixed, or the hydrogen gas may be added to a mixture solution containing at least one of or both the medium and the antifreezing agent.

As the hydrogen water, hydrogen water immediately after preparation may be used, or hydrogen water stored in a sealed container filled with hydrogen gas may be used.

Hydrogen gas may be added in the atmosphere or may be added under a pressure in the sealed space.

For freezing the cells, cooling may be performed at a mild condition of a cooling speed of about -1°C/min.

As for preservation of the cells, the cells may be stored in an environment at -80°C or less, preferably at -130°C or less, or more preferably -150°C or less, for example. For example, the cells can be stored using a liquid nitrogen preservation container.

The cell culture method includes: thawing the frozen article obtained by the cell freezing method to obtain a thawed article; and culturing the cells contained in the obtained thawed article.

For dissolving the cells, the frozen article may be exposed to an environment at 0°C to 40°C, preferably at about 36°C to 37°C, for example, for 30 seconds to 10 minutes, for example, although it differs depending on types of the cell freezing composition and the cells used for the freezing. For example, the frozen article may be immersed in a water bath at about 37°C. It is thus possible to appropriately thaw the cells in the frozen article.

The thawed cells are cultured.

Culture conditions differ depending on the type of cells to be cultured, and the culture can be performed under conditions typically performed. Although not particularly limited, cells of mammalian may be cultured at 37°C in a 5% CO₂ atmosphere.

A cell freezing kit according to the present embodiment will be described.

The cell freezing kit contains the cell freezing composition to be used in the cell freezing method or the components used to prepare the cell freezing composition.

One cell freezing kit may include at least a preservation container containing hydrogen water.

The preservation container may contain hydrogen water in a liquid phase and may contain hydrogen gas in a gas phase. It is possible to keep the concentration of dissolved hydrogen in the hydrogen water at a specific level or more by filling the gas phase with hydrogen gas. The storage temperature may be 0°C to 30°C or preferably 23°C to 27°C, for example.

A lower limit of the concentration of dissolved hydrogen in hydrogen water measured at 25°C under an atmospheric pressure immediately after opening of the storage container is equal to or greater than 0.1 ppm, is preferably equal to or greater than 0.2 ppm, and is more preferably equal to or greater than 0.3 ppm, for example. On the other hand, an upper limit of the concentration of dissolved hydrogen in the hydrogen water is equal to or less than 10.0 ppm, is equal to or less than 5.0 ppm, is preferably equal to or less than 2.0 ppm, is equal to or less than 1.6 ppm, and may be equal to or less than 1.5 ppm, for example.

The storage container may be configured with a material with low hydrogen transmittance and low oxygen transmittance, and an aluminum bag may be used, for example.

A user can prepare the cell freezing composition using hydrogen water extracted from the storage container in the cell freezing kit. It is possible to enhance cell proliferation ability after thawing using this.

The preservation container in the cell freezing kit may contain at least a mixture solution of hydrogen water and a medium.

It is possible to enhance cell proliferation ability after thawing with the cell freezing composition containing hydrogen water if the hydrogen water is used as a mixture solution.

The cell freezing kit may contain a protocol for freezing the cells.

A freezing procedure and a freezing condition of cells using the cell freezing composition are described in the protocol.

Also, the cell freezing kit may further contain a protocol for thawing the cells.

Although embodiments of the present invention have been described above, these are illustrations of the present invention, and various configurations other than those described above can be employed. Moreover, the present invention is not limited to the aforementioned embodiments, modifications, improvements, and the like within a range in which the object of the present invention can be achieved are included in the present invention.

Hereinafter, examples of reference forms will be described.
1. A cell freezing composition including: a medium; an antifreezing agent; and hydrogen water.
2. The cell freezing composition according to 1, in which a redox potential of the cell freezing composition measured at 25°C under an atmospheric pressure is equal to or greater than -700 mV and equal to or less than -100 mV.
3. The cell freezing composition according to 1 or 2, in which concentration of dissolved hydrogen in the cell freezing composition measured at 25°C under an atmospheric pressure is equal to or greater than 0.1 ppm and equal to or less than 1.6 ppm.
4. The cell freezing composition according to any one of 1 to 3, in which pH of the cell freezing composition measured at 25°C under an atmospheric pressure is equal to or greater than 6.8 and equal to or less than 8.5.
5. The cell freezing composition according to any one of 1 to 4, in which a concentration of dissolved oxygen in the cell freezing composition measured at 25°C under an atmospheric pressure is equal to or less than 5.0 ppm.
6. The cell freezing composition according to any one of 1 to 5, in which the antifreezing agent contains one or two or more selected from the group consisting of dimethyl sulfoxide (DMSO), hydroxyethyl starch (HES), ethylene glycol (EG), and glycerol.
7. The cell freezing composition according to any one of 1 to 6, in which cells used in cell freezing include animal cells.
8. A cell freezing composition including: a medium; and an antifreezing agent, in which a redox potential of the cell freezing composition measured at 25°C under an atmospheric pressure is equal to or greater than -700 mV and equal to or less than -100 mV, and pH of the cell freezing composition measured at 25°C under an atmospheric pressure is equal to or greater than 6.8 and equal to or less than 8.5.
9. A cell freezing method including: preparing a mixture of the cell freezing composition according to any one of 1 to 8 and cells; and freezing the mixture to obtain a frozen article.
10. A cell culture method including: thawing a frozen article obtained by the cell freezing method according to 9 to obtain a thawed article; and culturing cells contained in the obtained thawed article.
11. A cell freezing kit including: a preservation container containing hydrogen water.
12. The cell freezing kit according to 11, in which the preservation container includes at least a mixture solution of the hydrogen water and the medium.
13. The cell freezing kit according to 11 or 12, further including: a protocol for freezing the cells.

### EXAMPLES

Although the present invention will be described below in detail with reference to examples, the present invention is not limited at all to the description of these examples.

### <Preparation of hydrogen water>

### (Hydrogen water A)

Hydrogen water A was prepared using distilled water at an environmental temperature of 25°C by using a hydrogen water generator (Ultra Fine GALF manufactured by IDEC, pressure dissolution method) .

The used distilled water had a concentration of dissolved hydrogen of 0.00 ppm and a concentration of dissolved oxygen of 8 to 9 ppm.

The hydrogen water A immediately after the preparation had a concentration of dissolved hydrogen of 1.5 ppm, a concentration of dissolved oxygen of 2 ppm, pH of 7.42, and ORP of -476 mV.

Note that there were substantially no variations in numerical values of the concentration of dissolved hydrogen, the concentration of dissolved oxygen, pH, and ORP in the hydrogen water A after elapse of 15 minutes immediately after the preparation.

The concentration of dissolved hydrogen was measured using a dissolved hydrogen meter (manufactured by Unisense, H₂ microsensor) at 25°C under an atmospheric pressure.

The dissolved oxygen was measured using a dissolved oxygen meter (manufactured by Central Kagaku Corporation, CGS-5) at 25°C under an atmospheric pressure.

pH and OPR were measured using a pH/ORP meter (manufactured by Toko Kagaku Kenkyusho, TPX-999Si) at 25°C under an atmospheric pressure.

### (Hydrogen water B)

The obtained hydrogen water A immediately after the preparation was sealed in an aluminum bag (manufactured by GL Sciences, Inc., CCK-1) and was stored under an atmospheric pressure at 25°C for 2 to 4 weeks in a state in which the back was filled with hydrogen gas. The hydrogen water immediately after being extracted from the aluminum bag was used as hydrogen water B.

The hydrogen water B immediately after opening after storage had a concentration of dissolved hydrogen of 1.5 ppm, a concentration of dissolved oxygen of 2 ppm, pH of 7.43, and ORP of -525 mV.

### <Preparation of cell freezing composition, cell freezing preservation, and thawing>

### (Example 1)

(1) Preparation of cell freezing composition Mouse-derived cells (manufactured by KAC Co., Ltd., MC3T3-E1, adherent cells) was suspended in a culture medium (α-MEM medium) to obtain concentration of 5 × 10⁵ cells/mL, and thereby preparing a cell solution in a sterilized 15 mL conical tube.

The hydrogen water A immediately after preparation was added to 100 µL of obtained cell solution to adjust the concentration of dissolved hydrogen, a sterilized α-MEM medium (manufactured by Gibco, product name: Mem alpha basic (powder)) mixed thereinto, and 900 µL of mixture solution with concentration of dissolved hydrogen in Table 1 was obtained inside the sterilized 15 mL conical tube.

700 µL of obtained mixture solution, 200 µL of fetal bovine serum (FCS, manufactured by GE Healthcare, product name: High Clone FCS Bovine Serum), and 100 µL of dimethyl sulfoxide (DMSO, manufactured by NAKARAI TESQUE, antifreezing agent) were placed in a cryotube with a volume of 1 mL and were then mixed, thereby preparing a cell preservation solution (cell freezing composition) before freezing. Similarly, six microtubes filled with cell suspensions containing cells in cell preservation solutions were prepared.

### (2) Cell freezing operation

Each cryotube was cooled to -80°C at -1°C/min using a cell freezing container (manufactured by Cosmo Bio, Mr. Frosty) and was then cooled and frozen to -150°C or less in a preservation container containing liquid nitrogen. After checking the freezing, each microtube was stored for 2 weeks in a preservation container containing liquid nitrogen.

### (3) Cell thawing operation

Each cryotube was extracted from the preservation container and was placed in a constant temperature tank at 37°C for 3 minutes, and thawing of the cell preservation solution was checked.

### (4) Activity evaluation and proliferation evaluation

The cell suspensions containing the cells in the cell preservation solutions frozen, preserved, and thawed through the operations in (1) to (3) were used as evaluation target samples. Six samples were obtained from each of the six cryotubes.

The following MTT assay was conducted on each obtained sample, and a change in cell proliferation in 7 days after the thawing was evaluated.

Note that the aforementioned operations were performed in an environment at 25°C under an atmospheric pressure unless particularly mentioned otherwise.

The concentration of dissolved hydrogen, pH, and ORP were measured for each of the mixture solution immediately after the preparation, the cell preservation solution before freezing immediately after the preparation, and cell preservation solution after the freezing immediately after the thawing were measured. The results are shown in Table 1.

### (Comparative Example 1)

Samples were obtained similarly to Example 1 other than that sterilized water was used instead of the hydrogen water A and a mixture solution of the sterilized water and an α-MEM medium was used.

The used sterilized water had concentration of dissolved hydrogen of 0.00 ppm and concentration of dissolved oxygen of 8 to 9 ppm.

### (Example 2)

Samples were obtained similarly to Example 1 other than that the hydrogen water B stored in an aluminum bag was used instead of the hydrogen water A.

**[Table 1]**

| | Comparative Example 1 | | | Example 1 | | | Example 2 | | |
|---|---|---|---|---|---|---|---|---|---|
| | Concentration of dissolved hydrogen (ppm) | pH | ORP (mV) | Concentration of dissolved hydrogen (ppm) | pH | ORP (mV) | Concentration of dissolved hydrogen (ppm) | pH | ORP (mV) |
| Hydrogen water | - | | | 1.5 | 7.42 | -476 | 1.5 | 7.43 | -525 |
| Mixture solution | 0.0 | 8.41 | 92 | 1.3 | 8.50 | -588 | 1.3 | 8.43 | -653 |
| Cell preservation solution before freezing | 0.0 | 8.22 | 78 | 1.0 | 8.26 | -314 | 1.0 | 8.21 | -472 |
| Cell preservation solution after thawing | 0.0 | 8.30 | 36 | 0.0 | 8.38 | 23 | 0.0 | 8.30 | 66 |

### <MTT assay>

An MTT activity value per number of cells was measured in accordance with the following procedure.

The samples that were measurement targets obtained in the examples and the comparative examples were used to add the culture medium (α-MEM medium) to obtain the cell concentration of 5.0 × 10⁵ cells/mL to 1.5 × 10⁶ cells/mL and were mixed, thereby preparing solutions.

100 µL of mixture solution was seeded in each of 96 wells in a 96-well plate (manufactured by Corning Incorporated) and was cultured for 30 minutes in a CO₂ incubator under conditions of 37°C and concentration of carbon dioxide of 5%.

10 µL of mixture solution of WST-8 and 1-methoxy PMS was added to each well.

Subsequently, the mixture solution was cultured for 60 minutes in a CO₂ incubator under conditions of 37°C and the concentration of carbon dioxide of 5%.

After the culture ended, absorbance of 450 nm was measured using a microplate reader (manufactured by Molecular Devices, LLC., product name: SpectraMax i3). The obtained value of absorbance was defined as a cell survival activity (MTT activity value) of metabolism (respiration) of cells.

MTT activity values of the six samples were similarly measured.

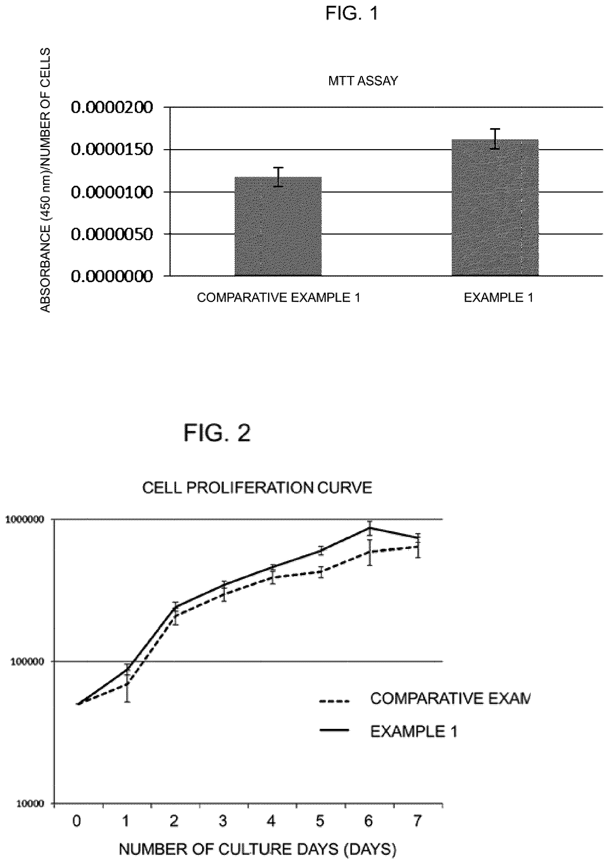

The MTT activity values per number of cells were calculated by dividing the obtained absorbance by used numbers of cells. Fig. 1 illustrates MTT activity values (average values of the six samples) per numbers of cells in Example 1 and Comparative Example 1.

Note that a hemocytometer was used to measure the numbers of cells.

### <Cell proliferation>

The samples that are measurement targets obtained in the examples and the comparative examples were used to culture the cells in accordance with the following procedure, and the numbers of cells by the day 7 of culture were measured with time.

Fig. 2 illustrates cell proliferation curves representing relationships between the number of cells (average values of 6 samples) and the numbers of culture days in Example 1 and Comparative Example 1.

The thawed cell preservation solutions were transferred to conical tubes and were centrifuged at 1000 rpm for 3 minutes, and supernatants were discarded. Thereafter, the cell preservation solutions were suspended in the culture medium (α-MEM medium) to obtain 5.0 × 10⁵ cells/mL. 10 mL of culture medium and each cell suspension were mixed on a separately prepared culture dish with a diameter of 100 mm.

Thereafter, culture was performed in a CO₂ incubator under conditions of 37°C and concentration of carbon dioxide of 5%. After the culture, the cells were peeled off from the culture dish and were then collected. The number of collected cells was measured using a hemocytometer.

A result that a high MTT activity value of cells after the thawing was achieved and the cell proliferation rate increased by using the cell freezing composition containing the hydrogen water in Example 1 as compared with Comparative Example 1 was shown. A trend similar to that in Example 1 was shown in Example 2 as well.

Therefore, it was discovered that the cell proliferation rate of the cells frozen and preserved using the cell freezing composition after the thawing increased regardless of whether or not the hydrogen water in Examples 1 and 2 was previously prepared.

### (Examples 3 to 5 and Comparative Example 2)

### (1') Preparation of cell freezing composition

Mouse-derived cells (manufactured by KAC Co., Ltd., MC3T3-E1, adherent cells) was suspended in a culture medium (α-MEM medium) to obtain concentration of 5 × 10⁵ cells/mL, and thereby preparing a cell solution in a sterilized 15 mL conical tube.

Separately, hydrogen gas was pressure-dissolved in an α-MEM medium, and a hydrogen-containing medium was prepared.

The hydrogen-containing medium and an ordinary α-MEM medium were added to 100 µL of obtained cell solution, thereby obtaining a mixture solution with the concentration of dissolved hydrogen adjusted to predetermined concentration.

In Examples 3 and 4, hydrogen gas was added to the medium in atmosphere, thereby preparing a hydrogen-containing medium.

In Example 5, the medium was sealed in an aluminum bag (manufactured by GL Sciences, Inc., CCK-1), hydrogen gas was added thereto in a pressurized state at 0.04 to 0.2 MPa, and the medium was shaken for 3 to 5 minutes or more, thereby preparing a hydrogen-containing medium in a state in which the dissolved hydrogen was supersaturated.

In Comparative Example 2, no hydrogen gas was added, in other words, no hydrogen-containing medium was used.

700 µL of obtained mixture solution, 200 µL of fetal bovine serum (FCS, manufactured by GE Healthcare, product name: High Clone FCS Bovine Serum), and 100 µL of dimethyl sulfoxide (DMSO, manufactured by NAKARAI TESQUE, antifreezing agent) were placed in a cryotube with a volume of 1 mL and were then mixed, thereby preparing a cell preservation solution (cell freezing composition) before freezing with the concentration of dissolved hydrogen in Table 2.

Similarly, six microtubes filled with cell suspensions containing cells in cell preservation solutions were prepared.

The concentration of dissolved hydrogen was measured using a dissolved hydrogen meter (manufactured by Unisense, H₂ microsensor) at 25°C under an atmospheric pressure.

### (2) Cell freezing operation

Each cryotube was cooled to -80°C at -1°C/min using a cell freezing container (manufactured by Cosmo Bio, Mr. Frosty) and was then cooled and frozen to -150°C or less in a preservation container containing liquid nitrogen. After checking the freezing, each microtube was stored for 2 weeks in a preservation container containing liquid nitrogen.

### (3) Cell thawing operation

Each cryotube was extracted from the preservation container and was placed in a constant temperature tank at 37°C for 3 minutes, and thawing of the cell preservation solution was checked.

### (4) Activity evaluation and proliferation evaluation

Cell suspensions containing cells in cell preservation solution frozen, preserved, and thawed through the aforementioned operations (1') to (3') were used as evaluation target samples. Six samples were obtained from each of the six cryotubes.

Note that in Comparative Example 2, the samples (reference examples) on which the freezing in (2) and (3) described above was not performed were also prepared.

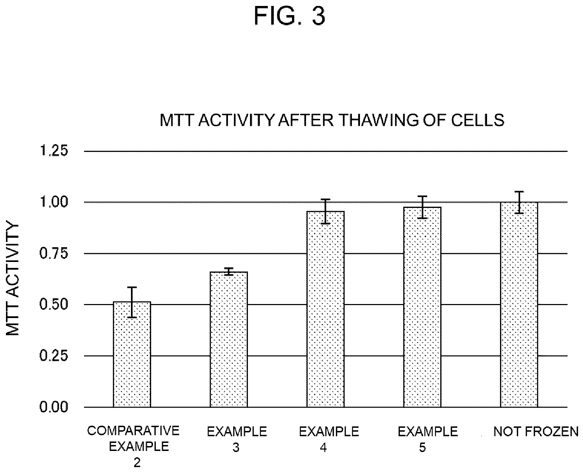

The aforementioned <MTT assay> was evaluated for each obtained sample. The results are shown in Table 3 and Fig. 3. For the MTT activity value per number of cells, the samples "that had not been frozen" (reference examples) were standardized at 1.00.

**[Table 2]**

| | Comparative Example 2 | Example 3 | Example 4 | Example 5 | Reference Example Not frozen |
|---|---|---|---|---|---|
| Concentration of dissolved hydrogen (ppm) | 0.0 | 0.5 | 1.0 | 2.0 | 0.0 |
| MMT activity value per number of cells | 0.51 | 0.66 | 0.95 | 0.98 | 1.00 |
| Standard deviation | 0.07 | 0.02 | 0.06 | 0.05 | 0.05 |

According to the cell freezing preservation method using the cell freezing composition in Examples 3 to 5, it was possible to obtain results of high MTT activity values of the cells after the thawing as compared with Comparative Example 2.

Results that higher MTT activity values of cells after the thawing were obtained in Examples 1 and 2 above as compared with Comparative Example 1 and in Examples 3 to 5 above as compared with Comparative Example 2 even in a case in which mouse-derived cells (RAW 264.7) or human-derived cells (THP-1) were used instead of the mouse-derived cells (MC3T3-E1).

The present application claims priority based on Japanese Patent Application No. 2019-185161 filed October 8, 2019, entire disclosures of which are incorporated herein.

## Claims

1. A cell freezing composition comprising:
a medium;
an antifreezing agent; and
dissolved hydrogen.

2. The cell freezing composition according to claim 1, further comprising:
hydrogen water.

3. The cell freezing composition according to claim 1 or 2, wherein a concentration of the dissolved hydrogen in the cell freezing composition measured at 25°C under an atmospheric pressure is equal to or greater than 0.1 ppm and equal to or less than 10.0 ppm.

4. The cell freezing composition according to any one of claims 1 to 3, wherein a redox potential of the cell freezing composition measured at 25°C under an atmospheric pressure is equal to or greater than -700 mV and equal to or less than -100 mV.

5. The cell freezing composition according to any one of claims 1 to 4, wherein pH of the cell freezing composition measured at 25°C under an atmospheric pressure is equal to or greater than 6.8 and equal to or less than 8.5.

6. The cell freezing composition according to any one of claim 1 to 5, wherein a concentration of dissolved oxygen in the cell freezing composition measured at 25°C under an atmospheric pressure is equal to or less than 5.0 ppm.

7. The cell freezing composition according to any one of claims 1 to 6, wherein the antifreezing agent includes one or more selected from the group consisting of dimethyl sulfoxide (DMSO), hydroxyethyl starch (HES), ethylene glycol (EG), and glycerol.

8. The cell freezing composition according to any one of claims 1 to 7, wherein cells used in cell freezing include animal cells.

9. A cell freezing composition comprising:
a medium; and
an antifreezing agent,
wherein a redox potential of the cell freezing composition measured at 25°C under an atmospheric pressure is equal to or greater than -700 mV and equal to or less than -100 mV, and pH of the cell freezing composition measured at 25°C under an atmospheric pressure is equal to or greater than 6.8 and equal to or less than 8.5.

10. A cell freezing method comprising:
preparing a mixture of a cell freezing composition that includes a medium, an antifreezing agent, and dissolved hydrogen and cells; and
freezing the mixture to obtain a frozen article.

11. The cell freezing method according to claim 10, wherein as a method for preparing the cell freezing composition, at least one of a method of mixing hydrogen water into the medium, a method of mixing hydrogen water into the antifreezing agent, a method of adding hydrogen water to a mixture solution containing the medium and the antifreezing agent, a method of adding hydrogen gas to the medium, a method of adding hydrogen gas to the antifreezing agent, and a method of adding hydrogen gas to a mixture solution containing the medium and the antifreezing agent.

12. A cell culture method comprising:
thawing the frozen article obtained by the cell freezing method according to claim 10 or 11 to obtain a thawed article; and
culturing the cells contained in the obtained thawed article.

13. A cell freezing kit comprising:
a preservation container containing hydrogen water.

14. The cell freezing kit according to claim 13, wherein the preservation container contains a mixture solution of at least the hydrogen water and the medium.

15. The cell freezing kit according to claim 13 or 14, further comprising a protocol for freezing cells.
